(19) Europäisches Patentamt
European Patent Office
Office européen des brevets

(11) Veröffentlichungsnummer: **0 037 585**
**A2**

(12) # EUROPÄISCHE PATENTANMELDUNG

(21) Anmeldenummer: 81102652.5

(22) Anmeldetag: 08.04.81

(51) Int. Cl.³: C 07 D 207/408
C 07 D 209/52, A 01 N 43/36
A 01 N 43/38

(30) Priorität: 09.04.80 DE 3013566

(43) Veröffentlichungstag der Anmeldung:
14.10.81 Patentblatt 81/41

(84) Benannte Vertragsstaaten:
AT BE CH DE FR GB IT LI NL SE

(71) Anmelder: Consortium für elektrochemische Industrie GmbH
Zielstattstrasse 20
D-8000 München 70(DE)

(72) Erfinder: Häberle, Norman, Dr., Dipl.-Chem.
Willibaldstrasse 51a
D-8000 München 21(DE)

(72) Erfinder: Eberle, Otto, Dr., Dipl.-Chem.
Burgmeierstrasse 15
D-8012 Ottobrunn(DE)

(72) Erfinder: Hafner, Walter, Dr., Dipl.-Chem.
Sommerfeld 15
D-8024 Furth(DE)

(54) **Herstellung von N-Phenyl-Bernsteinsäureimiden und ihre Verwendung als fungizide Mittel.**

(57) Die Erfindung betrifft Verbindungen der allgemeinen Formel

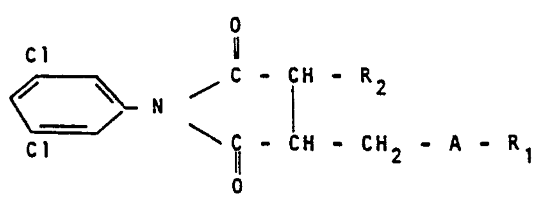

und die Verbindung der allgemeinen Formel

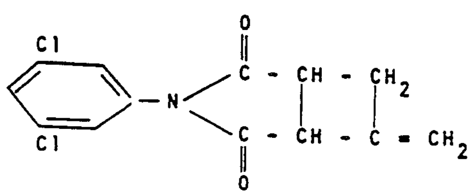

wobei

A die -Ch = CH - oder die - C = C-Gruppe bedeutet und $R_1$ und $R_2$ für Wasserstoff oder Methyl stehen. Diese Verbindungen weisen fungizide Wirksamkeit auf.

Die erfindungsgemäßen Wirkstoffe sind durch Umsetzung entsprechend substituierter Bernsteinsäuren sowie deren Säurehalogeniden, -anhydriden oder -estern mit 3.5 Dichloranilin zugänglich.

EP 0 037 585 A2

Consortium
für elektrochemische Industrie GmbH

München, den 3.4.1980
LC-PAT/Dr.Ra/we

Co 8002

## N-(3.5-Dichlorphenyl)-Bernsteinsäureimide, deren Herstellung und ihre Verwendung als fungizide Mittel

Aus der Reihe der N-(3.5-Dichlorphenyl)-Succinimide sind bereits Verbindungen bekanntgeworden, die fungizide Eigenschaften aufweisen.

Eine zusammenfassende Darstellung über dieses Thema findet sich in R. Wegler, Chemie der Pflanzenschutz- und Schädlingsbekämpfungsmittel, Band 4, Seite 201 ff., Springer-Verlag, 1977.

In der europäischen Patentanmeldung 000 1395 werden ebenfalls fungizide N-(3.5-Dichlorphenyl)-Succinimide beschrieben, die insbesondere cycloaliphatische Substituenten, aber auch z.B. den Isobutenylrest oder verzweigte Alkylgruppen am Bernsteinsäuregerüst tragen.

Ferner werden in der DE-OS 19 40 032 fungizide N-(3.5-Dichlorphenyl)-Succinimide angegeben mit aromatischen, aber auch kleineren gesättigten aliphatischen Substituenten am Bernsteinsäurerest.

Weiterhin wurden in der DE-OS 20 12 656 eine Reihe von hochsubstituierten N-(3.5-Dichlorphenyl)-Succinimiden als Mikrobizide veröffentlicht, insbesondere solche, die Heteroatome im Substitutionsteil besitzen.

In der DE-OS 15 42 835 werden Verbindungen dieser Art auch als Herbizide beschrieben.

Es ist also gemäß dem Stand der Technik bekannt, daß Bernsteinsäureimide des 3.5-Dichloranilins mehr oder minder fungitoxisch Eigenschaften besitzen.

Aufgabe der Erfindung war es nun, aus dieser Klasse von Verbindungen solche zu finden, die nach folgenden Kriterien besondere Vorteile aufweisen:

1. Gute Zugänglichkeit bezüglich des chemischen Synthesewegs
2. Hohe Fungitoxizität, d.h. hohe Wirksamkeit bei kleinsten Dosen
3. Breites Wirkungsspektrum
4. Gute Verträglichkeit für höhere Pflanzen
5. Unschädlichkeit gegenüber höheren Lebewesen.

Es wurde gefunden, daß diese oben zitierten Kriterien in besonderem Maße von einer kleinen Auswahl von Bernsteinsäureimiden des 3.5-Dichloranilins erfüllt werden.

Gegenstand der Erfindung sind Verbindungen der allgemeinen Formel

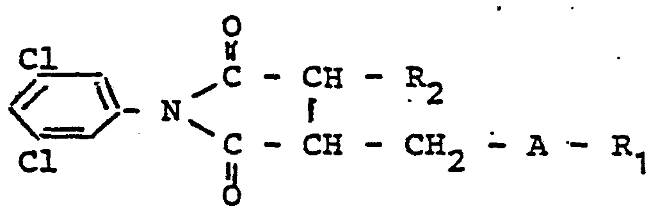

und die Verbindung der Formel

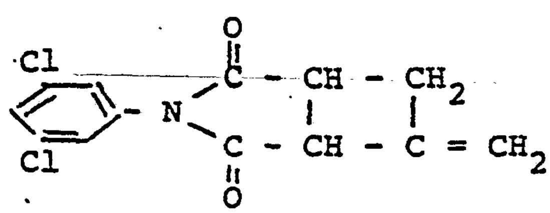

wobei
A die $-CH = CH-$ oder die $-C \equiv C$-Gruppe bedeutet,
$R_1$ für H- oder $CH_3$-, steht, und
$R_2$ H- oder $CH_3$- sein kann.

Den erfindungsgemäßen Verbindungen ist zu eigen, daß sie Substituenten besitzen, mit einer Kohlenstoffmehrfachbindung, die nicht in Konjugation mit der Carbonylfunktion des Bernsteinsäurerests stehen. Überraschenderweise zeigen Verbindungen, die dieses Merkmal in Kombination mit einer bestimmten Kettenlänge des Substituenten besitzen, nämlich mit 3 bis 4 Kettengliedern, besonders günstige Eigenschaften.

Beispiele für erfindungsgemäße Verbindungen sind

2-Allyl-Bernsteinsäure-N-(3.5-Dichlorphenyl)-imid
2-Allyl-3-Methyl-Bernsteinsäure-N-(3.5-Dichlorphenyl)-imid
2-Propargyl-Bernsteinsäure-N-(3.5-Dichlorphenyl)-imid
2-Propargyl-3-Methyl-Bernsteinsäure-N-(3.5-Dichlorphenyl)-imid
2-(But-2-en-1-yl)-Bernsteinsäure-N-(3.5-Dichlorphenyl)-imid
2-(But-2-en-1-yl)-3-Methyl-Bernsteinsäure-N-(3.5-Dichlorphenyl)-imid
2-(But-2-in-1-yl)-Bernsteinsäure-N-(3.5-Dichlorphenyl)-imid
2-But-2-in-1-yl)-3-Methyl-Bernsteinsäure-N-(3.5-Dichlorphenyl)-imid
3-Methylencyclobutan-1.2-Dicarbonsäure-N-(3.5-Dichlorphenyl)-imid

Weiterhin sind Gegenstand der Erfindung fungizid wirksame Zusammensetzungen, die zumindest eine der erfindungsgemäßen Verbindungen enthalten.

Die erfindungsgemäßen fungiziden Mittel eignen sich, ohne daß ihr Anwendungsbereich etwa darauf beschränkt wäre, z.B. zum Einsatz im Weinbau, in Erdbeerpflanzungen, im Gartenbau, insbesondere in Salatpflanzungen, im Rapsanbau oder bei Zierpflanzen (Alpenveilchen, Geranien u.a.). Als weitere erfindungsgemäße Anwendung wurde der Einsatz als Saatgutbeizmittel gefunden.

Die erfindungsgemäßen Wirkstoffe bewähren sich bei der Bekämpfung der pilzlichen Krankheiten. Sie erweisen sich z.B. als hochwirksam gegen Botrytis cinerea (Grauschimmel). Weitere Beispiele sind Pilze wie Alternaria solani, Fusarium nivale, Septoria nodorum, Verticillium dahliae, Penicillium glaucum, die Sclerotinia Arten (z. B. Sclerotinia Sclerotiorum), Phomalingam und andere. Weiter sind die erfindungsgemäßen Wirkstoffe erfolgreich gegen phytopathogene Pilze, die dem Saatgut anhaften, einsetzbar, wie z. B. Tilletia tritici (Weizensteinbrand).
Der Bedarf an solchen neuen Fungiziden begründet sich nicht allein auf ihre hervorragende Wirksamkeit, sondern darüberhinaus auf der Eigenschaft vieler Pilzarten, gegen bereits angewandte Fungizide resistente Stämme zu bilden.

Ein besonderer Vorteil der erfindungsgemäß anzuwendenden Wirkstoffe besteht weiterhin in ihrer physiologischen Unbedenklichkeit gegenüber höheren Lebewesen. Da Fungizide häufig bei Nahrungsmitteln zum Einsatz gelangen, ist gerade diese Eigenschaft für die moderne Landwirtschaft von hohem Wert.

Die erfindungsgemäßen Bernsteinsäureimide lassen sich in grundsätzlich bekannter Weise darstellen. Das Verfahren ist dadurch gekennzeichnet, daß die entsprechend substituierte Bernstein-

säure, deren Säurehalogenid oder Säureanhydrid oder ein Säureester, ggf. in Gegenwart von tertiären organischen Basen, wie z.B. Pyridin oder Triäthylamin, mit 3.5-Dichloranilin umgesetzt wird.

Es kann unter Umständen günstig sein, zur Reaktion wasser- oder säurebindende Mittel, wie z.B. Alkali- oder Erdalkalicarbonate, Alkali- oder Erdalkalihydroxide oder -oxide, insbesondere Soda, Pottasche, Magnesia, gebrannten, gelöschten oder kohlensauren Kalk einzusetzen.

Die benötigten substituierten Bernsteinsäurederivate können, soweit sie nicht ohnehin im Handel erhältlich sind, nach bekannten Verfahren hergestellt werden. Die Verfahren sind dadurch gekennzeichnet, daß entsprechende Olefine oder Acetylene, ggf. in geeigneten Druckgefäßen, mit Maleinsäureanhydrid umgesetzt werden. Die Darstellung von Methylencyclobutan-Bernsteinsäure erfolgt in analoger Weise durch Verwendung von Allen.

Die erfindungsgemäßen Wirkstoffe können allein oder im Gemisch mit sonstigen geeigneten Pflanzenschutzmitteln ausgebracht werden. Im allgemeinen werden sie jedoch als Mischungen mit festen oder flüssigen Verdünnungsmitteln oder als Lösungen in festen oder flüssigen Lösungsmitteln verwendet, mit Wirkstoffgehalten von 0,01 bis 95 Gew.%.

Die Mischungen bzw. Lösungen werden im allgemeinen als Emulsionskonzentrate, Pasten, Spritzpulver, Granulate oder Mikrokapseln hergestellt.

Emulsionskonzentrate und Pasten enthalten im allgemeinen 10 bis 60 Gew.%, vorzugsweise 15 bis 40 Gew.% Wirkstoff, 2 bis 25 Gew.% Dispergierhilfsstoffe und organische Lösungsmittel und/oder Wasser.

Spritzpulver enthalten meistens 10 bis 80 Gew.%, vorzugsweise 15 bis 70 Gew.% Wirkstoff, 1 bis 10 Gew.% Dispergierhilfsstoffe und 10 bis 89 Gew.% inerte Bestandteile.

Granulate und Stäubemittel enthalten neben inerten Bestandteilen, Bindemitteln und/oder Überzugsstoffen 1 bis 10 Gew.%, vorzugsweise 5 bis 10 Gew.% Wirkstoff.

Erfindungsgemäß angewandt werden:

Als Dispergierhilfsstoffe z.B.
Alkyl- und Arylsulfonate, Methylcellulose, polymere Sulfonsäuren und deren Salze, Polyalkohole, Fettsäureester, Fettalkoholäther, Fettamine;

als organische Lösungsmittel z.B.
Alkohole, wie Äthanol, Butanole, Dimethylformamid, Dimethylsulfoxid, N-Methylpyrrolidon, Aromaten, wie Toluol und Xylole;

als inerte Bestandteile z.B.
Kaolin, China-Clay, Talkum, Calciumcarbonat, hochdisperse Kieselsäure, Kieselgele, Kieselgur, Diatomenerde, Bims, Maisschrot, Verdickungsmittel, wie Stärke und Carboxymethylcellulose;

als Bindemittel z.B.
Magnesiumsulfat, Gips, Gummiarabikum.

Beispielsweise werden die erfindungsgemäßen Fungizide wie folgt formuliert:

1. Emulsionskonzentrat:
   20 Gew.% Wirkstoff
   10 Gew.% handelsübliches äthoxyliertes Anhydrosorbitmonolaurat (Handelsname "Tween Twenty")
   70 Gew.% Dimethylformamid

2. Spritzpulver:

20 Gew.% Wirkstoff
5 Gew.% Ammoniumligninsulfonat (Handelsname "Totanin")
10 Gew.% Natriumoleylmethyltaurid (Handelsname "Arkopon T Konz")
65 Gew.% Kaolin

Die Aufwandmengen an Wirkstoffen können in großen Bereichen variieren. Bei der Saatgutbehandlung werden im allgemeinen Wirkstoffmengen von 0,05 bis 25 g/kg Saatgut benötigt.

Die Ausbringung der erfindungsgemäßen Wirkstoffe kann in jeder geeigneten Form erfolgen, beispielhaft genannt seien: Gießen, Verspritzen, Versprühen, Verstreuen, Bestreichen, Behandeln des Saatgutes (Beizen).

Im folgenden werden die Darstellungsmethoden der erfindungsgemäß anzuwendenden Verbindungen und ihre fungizide Wirksamkeit anhand von Beispielen erläutert:

Beispiel 1

Darstellung von 2-Allylbernsteinsäure-N-(3.5-Dichlorphenyl)-imid

28 g (0,2 Mol) Allylbernsteinsäureanhydrid, 32,4 g (0,2 Mol) 3.5-Dichloranilin, 0,6 g Triäthylamin und 150 ml Xylol werden bei 125° C unter Rühren umgesetzt. Das entstehende Reaktionswasser wird über einen Wasserabscheider aceotrop entfernt. Die Reaktionsdauer beträgt 4 Stunden. Anschließend wird das Lösungsmittel abgezogen und der Rückstand aus Essigester unter Zusatz von Aktivkohle umkristallisiert. Das Produkt besitzt einen Schmelzpunkt von 121° C. Die Ausbeute beträgt 89,4 % der Theorie.

Beispiel 2

Darstellung von 2-Allyl-3-Methylbernsteinsäure-N-(3.5-Dichlorphenyl)-imid

90,5 g (0,5 Mol) 2-Allylmalonsäurediäthylester werden in Diäthylenglykoldimethyläther mit 0,5 Mol alkoholfreiem Natriumäthylat umgesetzt. Zu dieser Lösung kommen unter Rühren tropfenweise 0,5 Mol 2-Brompropionsäureäthylester. Das Gemisch wird noch 2 Stunden am Rückfluß erhitzt, danach das Lösungsmittel abgezogen. Der Rückstand wird anschließend in alkoholischer KOH der Hydrolyse unterworfen. Die sich abtrennende organische Schicht wird nach Trocknung fraktioniert. Der gesuchte 2-Allyl-1-Methyläthantricarbonsäure-1.2.2-Triäthylester siedet unter 0.02 Torr bei 92 bis 95° C. Der Ester wird in alkoholischer KOH-Lösung verseift und nach üblicher Aufarbeitung bei 170° C unter Wasserabspaltung decarboxiliert. Es wird in 96 %-iger Ausbeute (bezogen auf den Ester) das Anhydrid der 2-Allyl-3-Methylbernsteinsäure ($K_p$ bei 0,1 Torr: 108 bis 110° C) erhalten.

Das Anhydrid wird mit 3.5-Dichloranilin, wie unter Beispiel 1 beschrieben, umgesetzt.

Das gewünschte Produkt weist einen Schmelzpunkt von 131° C auf.

Beispiel 3

3-Methylencyclobutan-1.2-Dicarbonsäure-N-(3.5-Dichlorphenyl)-imid

In einem 2-Liter Stahlautoklaven mit Rührer werden 500 g (5,1 Mol) Maleinsäureanhydrid, 645 ml Benzol und 0,25 g Hydrochinon unter Rühren auf - 70° C abgekühlt. Nach Evakuieren auf 20 Torr werden 100 g (2,5 Mol) Allen in den Autoklaven eingesaugt. Das Reaktionsgemisch wird anschließend unter Rühren auf 200° C erhitzt. Nach 8 Stunden wird auf 25° C abgekühlt, entspannt und nicht umgesetztes Allen in einer Kühlfalle gesammelt. Die benzolische Lösung wird abdekantiert, der verbleibende Rückstand in 500 ml Aceton gelöst. Die vereinigten benzolischen und acetonischen Lösungen werden anschließend destillativ aufgearbeitet. Nach Abzug der Lösungsmittel wird zwischen 110 bis 115° C unter einem Druck von 40 Torr bis zu 250 g Maleinsäureanhydrid zurückgewonnen. Schließlich werden 119 g Rohprodukt an gewünschtem Anhydrid unter 3 Torr in einem Siedeintervall von 70 bis 125° C erhalten. Das Rohprodukt wird anschließend unter 25 Torr bei 155 bis 159° C rektifiziert. Die Ausbeute beträgt 25 %.

Das Anhydrid wird, wie unter Beispiel 1 beschrieben, mit 3.5-Dichloranilin umgesetzt. Das erhaltene Produkt weist einen Schmelzpunkt von 119° C auf.

Beispiel 4

Traubensafttest

20 ml einer Nährlösung aus Traubensaft und destilliertem Wasser im Gewichtsverhältnis 1 : 1 werden in Glas-Petrischalen eingefüllt und mit den in der Tabelle 1 angeführten Wirkstoffen in den angegebenen Konzentrationen versetzt. Anschließend werden die Versuchsansätze mit jeweils 50 µl einer Botrytis-Sporensuspension, hergestellt durch Abschwemmen der Botrytis-Sporen von einer Agarkultur mit destilliertem Wasser, beimpft.

Nach einer Bebrütungsdauer von 10 bzw. 20 Tagen bei 20° C wird das Ausmaß der Pilzentwicklung auf der Nährlösungsoberfläche beurteilt.

Der Wirkungsgrad wird in Prozent nach der folgenden Formel errechnet:

$$100 - \frac{\text{Pilzwachstum, behandelt}}{\text{Pilzwachstum, unbehandelt}} \times 100$$

Tabelle 1

Wirksamkeit gegen Botrytis cinerea im Traubensafttest bei 10 ppm Wirkstoffkonzentration

| Wirkstoff | % Wirksamkeit nach 10 Tagen | % Wirksamkeit nach 20 Tagen |
|---|---|---|
| 2-Allylbernsteinsäure-N-(3.5-Dichlorphenyl)-imid | 100 | 100 |
| 2-(But-2-en-1-yl) Bernsteinsäure-N-(3.5-Dichlorphenyl)-imid | 100 | 100 |
| 2-Propargylbernsteinsäure-N-(3.5-Dichlorphenyl)-imid | 100 | 100 |
| 3-Methylencyclobutan-1.2-Dicarbonsäure-N-(3.5-Dichlorphenyl)-imid | 100 | 100 |

Vergleichsbeispiel 1

Versuchsanordnung und -durchführung sind die gleichen wie in Beispiel 4 beschrieben. Die Wirkstoffkonzentration ist jedoch im Gegensatz dazu 10 mal so hoch, nämlich 100 ppm.

Tabelle 2

Wirksamkeit gegen Botrytis cinerea im Traubensafttest bei 100 ppm Wirkstoffkonzentration

| Wirkstoff | % Wirksamkeit nach 10 Tagen | % Wirksamkeit nach 20 Tagen |
|---|---|---|
| 2-Allylbernsteinsäure-N-(3.4-Dichlorphenyl)-imid | 0 | 0 |

Beispiel 5

Sporenkeimtest

50 µl einer Lösung oder Suspension des Wirkstoffs mit einem Gehalt von 500 ppm Aktivsubstanz werden zusammen mit 50 µl einer Sporensuspension, hergestellt durch Abschwemmen der Sporen von einer Agarkultur mit einer Nährlösung, die pro Liter 10 g Zucker, 1 g Glykol, 1 g $KH_2PO_4$ und 0,5 g $MgSO_4$ enthält, in den Hohlschliff von Hohlschliffobjektträgern eingebracht.

Die Objektträger werden bei 20° C 48 Stunden in einer Petrischale, deren Boden mit einem angefeuchteten Filterpapier bedeckt ist, aufbewahrt.

Danach wird das Verhältnis der gekeimten und der nicht gekeimten Sporen gegen eine unbehandelte Kontrollprobe verglichen.

Der Wirkungsgrad wird in Prozent nach der folgenden Formel berechnet:

$$100 - \frac{\text{Anzahl der gekeimten Sporen, behandelt}}{\text{Anzahl der gekeimten Sporen, unbehandelt}} \times 100$$

Die Ergebnisse sind in der Tabelle 3 dargestellt.

08.04.81 M
0037585

<u>Vergleichsbeispiel 2</u>

Versuchsanordnung und -durchführung sind die gleichen, wie in Beispiel 5 beschrieben.

Die Ergebnisse sind in der Tabelle 4 dargestellt.

Tabelle 3

Fungitoxizität gegen Pilzsporen bei 500 ppm Wirkstoffkonzentration

| Wirkstoff | Alternaria solani | Botrytis cinerea | Fusarium nivale | Penicillium glaucum | Septoria nodorum |
|---|---|---|---|---|---|
| 2-Allylbernsteinsäure-N-(3.5-Dichlorphenyl)-imid | 80 | 100 | 60 | 80 | 60 |
| 2-(But-2-en-1-yl)-bernsteinsäure-N-(3.5-Dichlorphenyl)-imid | 70 | 80 | 80 | 80 | 60 |
| 2-Propargylbernsteinsäure-N-(3.5-Dichlorphenyl)-imid | 80 | 100 | 80 | 80 | 40 |
| 3-Methylencyclobutan-1.2-Dicarbonsäure-N-(3.5-Dichlorphenyl)-imid | 80 | 100 | 100 | 80 | 60 |

Tabelle 4

Fungitoxizität von Vergleichswirkstoffen gegen Pilzsporen bei 500 ppm Wirkstoffkonzentration

| Wirkstoff | Alternaria solani | Botrytis cinerea | Fusarium nivale | Penicillium glaucum | Septoria nodorum |
|---|---|---|---|---|---|
| 2-Allylbernsteinsäure-N-(3.4-Dichlorphenyl)-imid | 0 | 0 | 0 | 0 | 0 |
| 2-Cyclohexen-Bernsteinsäure-N-(3.5-Dichlorphenyl)-imid | 30 | 25 | 0 | 0 | 30 |
| 3-(3.5-Dichlorphenyl)-1-isopropyl-carbamoyl-hydantoin Handelsname Rovral | 0 | 100 | 0 | 60 | 30 |
| Vinchlozolin Handelsname Ronilan | 30 | 100 | 40 | 30 | 40 |

Aufschlußreich sind insbesondere Vergleiche der fungitoxischen Daten von 2-Allylbernsteinsäure-N-(3.5-Dichlorphenyl)-imid mit den Vergleichspräperaten aus Vergleichsbeispiel 1 und den beiden erstgenannten Bernsteinsäurederivaten aus Vergleichsbeispiel 2.

Die beiden Vergleichspräperate unterscheiden sich in einem Fall durch die Art der Chlorsubstitution an der Phenylgruppe (3.4 statt 3.5) und im anderen Fall durch die Substitution am Bernsteinsäurerest. Es zeigt sich, daß erst die Kombination der einzelnen Molekülbauteile - Bernsteinsäureimid + N-(3.5-Dichlorphenyl) + spezifische Auswahl des Substituenten am Bernsteinsäurerest - zu den überraschend hohen fungitoxischen Eigenschaften führt.

Weiterhin belegen die Ergebnisse aus Beispiel 5 und Vergleichsbeispiel 2 das verbreiterte Wirkungsspektrum der erfindungsgemäßen Verbindungen. Diese Eigenschaft ist oft von entscheidender Bedeutung: so treten z. B. neben Botrytis cinerea oft Begleitpilze, wie Alternaria solani und Penicillium glaucum auf. Richtet sich ein Fungizid nur gegen einen bestimmten Pilzstamm, so wird oft das verstärkte Aufwachsen der Begleitpilzarten beobachtet. Eine aussichtsreiche Behandlung einer Pilzkrankheit ist demnach erst dann gewährleistet, wenn das eingesetzte Fungizid ein derart verbreitertes Wirkungsspektrum besitzt, daß es den Gesamtkomplex der Pilzkrankheit bekämpft.

<u>Beispiel 6</u>

Saatgutbeizmitteltest

Gesundes Weizensaatgut wird mit Sporen von Tilletia tritici gleichmäßig infiziert. Anschließend wird das infizierte Saatgut mit den als Trockenbeizmittel formulierten erfindungsgemäßen Wirkstoffen sorgfältig gebeizt.

0037585

Nach dem Beizen werden die Weizenkörner in feuchte Erdschalen eingelegt. Die Bebrütung erfolgt bei 14 bis 17° C in einem dunklen Trockenschrank. Nach 48 Stunden werden die an den Körnern haftenden Sporen durch Eindrücken in Lehm/Erde abgetupft.

Nach weiterer 8-tägiger Bebrütung bei 14 bis 17° C im Trockenschrank wird unter dem Binokular das Verhältnis der gekeimten und der nicht gekeimten Sporen im Vergleich zu einer unbehandelten Kontrolle ermittelt.

Die Wirkung der erfindungsgemäßen Wirkstoffe wird in Prozent Keimverhinderung angegeben.

Tabelle 5

Beizmitteltest gegen Weizensteinbrand (Tilletia tritici)

| | Wirksamkeit % | |
|---|---|---|
| Wirkstoff | bei 1000 ppm Wirkstoff-konzentration | bei 100 ppm Wirkstoff-konzentration |
| 2-Allylbernsteinsäure-N-(3.5-Dichlorphenyl)-imid | 100 | 85 |
| 2-(But-2-en-1yl)-bernsteinsäure-N-(3.5-Dichlorphenyl)-imid | 100 | 85 |

Patentansprüche

1. Verbindungen der allgemeinen Formel

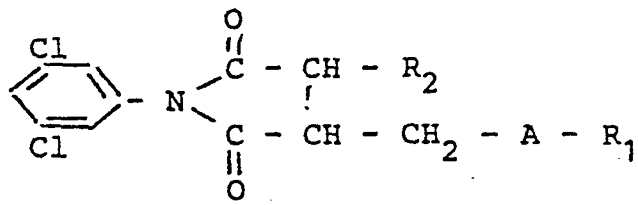

und die Verbindung der Formel

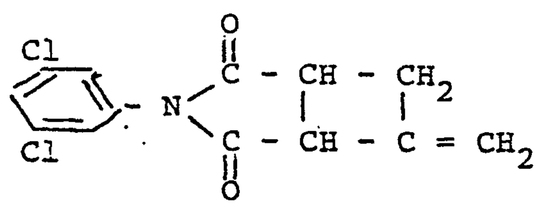

wobei

A die -Ch = CH- oder die -C ≡ C-Gruppe bedeutet,

$R_1$ für H- oder $CH_3$-, steht, und

$R_2$ H- oder $CH_3$- sein kann.

2. Fungizid wirksame Zusammensetzungen, die mindestens eine Verbindung nach Anspruch 1 enthalten.

3. Verfahren zur Herstellung von Verbindungen nach Anspruch 1, der allgemeinen Formel

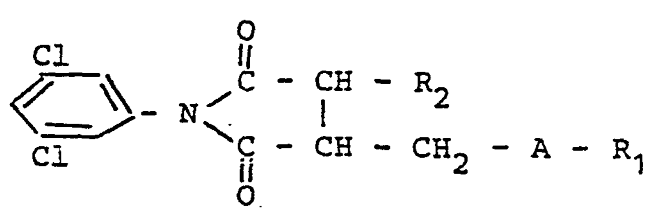

oder einer Verbindung der Formel

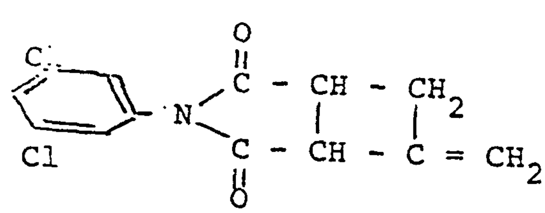

0037585

wobei A die -Ch = CH- oder die -C ≡ C-Gruppe bedeutet,

$R_1$ für H- oder $CH_3$- steht, und
$R_2$ H- oder $CH_3$ sein kann,

d a d u r c h g e k e n n z e i c h n e t , daß die entsprechend substituierte Bernsteinsäure, deren Säurehalogenid, Säureanhydrid oder Ester, ggf. in Gegenwart von tertiären organischen Basen, mit 3.5-Dichloranilin umgesetzt werden.